# EUROPÄISCHE PATENTANMELDUNG

(11) **EP 0 613 889 A2**
(43) Veröffentlichungstag der Anmeldung: **07.09.1994**
(21) Anmeldenummer: 93810880.0
(22) Anmeldetag: 14.12.1993
(51) Int. Cl.: C07D 233/84, C07D 235/28, C07D 249/12, C07D 513/14, B41M 5/28

(54) **Thermochrome Verbindungen deren Herstellung und deren Verwendung**

(30) Priorität: 23.12.1992 CH 3945/92
(71) Anmelder: CIBA-GEIGY AG, CH-4002 Basel (CH)
(72) Erfinder: Dr. Fischer, Walter, CH-4153 Reinach (CH); Dr. Schmidhalter, Beat, CH-6208 Oberkirch (CH); Dr.Wolleb, Heinz, CH-1723 Marly (CH)

(57) **Zusammenfassung**

Es werden Verbindungen der Formeln 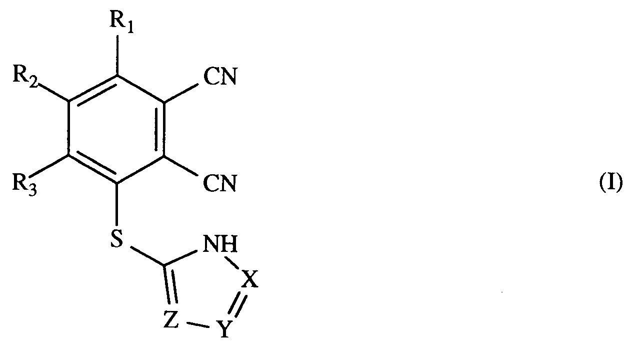
in welchen R₁, R₂ und R₃ unabhängig voneinander je Wasserstoff, eine C₁-C₁₂-Alkylgruppe, eine C₁-C₁₂-Alkoxygruppe, eine C₁-C₁₂-Alkylthiogruppe, eine C₅-C₈-Cycloalkylgruppe, eine C₅-C₈-Cycloalkoxygruppe, eine C₅-C₈-Cycloalkylthiogruppe, eine C₆-C₁₀-Arylgruppe, eine C₆-C₁₀-Aryloxygruppe, eine C₆-C₁₀ -Arylthiogruppe, Nitro, Halogen, Cyano, -COOH, -COOR₇, -NR₈R₉ oder -CONR₈R₉ bedeuten, wobei R₇ C₁-C₂₀-Alkyl, C₅-C₈-Cycloalkyl, Phenyl oder Phenyl-C₁-C₄-alkyl bedeutet, und R₈ und R₉ unabhängig voneinander je Wasserstoff, C₁-C₈-Alkyl, Phenyl oder Phenyl-C₁-C₄-alkyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, eine geradkettige oder verzweigte C₁-C₁₂-Alkylgruppe oder eine C₆-C₁₀ -Arylgruppe bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen auch eine Gruppe -CH=C(R₁₀)-C(R₁₁)=CH- oder eine Tetramethylengruppe bilden, in welcher R₁₀ und R₁₁ unabhängig voneinander Wasserstoff, C₁-C₆-Alkyl, Phenyl oder Phenyl-C₁-C₄-alkyl darstellen, offenbart. Die Verbindungen eignen sich als Aktivbestandteile in thermischen Informationsaufzeichnungsverfahren, besonders für laseroptische Informationsspeicher.

## Beschreibung

Die vorliegende Erfindung betrifft neue thermochrome Verbindungen, Verfahren zu ihrer Herstellung, ihre Verwendung in thermochromen Systemen zur Kontrastbildung, zur Lichtabsorption und zur Aufzeichnug von Informationen, sowie thermochrome Aufzeichnugsmaterialien, welche die neuen Verbindungen als thermochrome Wirkstoffe enthalten.

Laseroptische Informationsspeicher in Form von CD-kompatiblen, einmal beschreibbaren und beliebig oft auslesbaren Informationen (WORM-Systeme) sind zum Beispiel in der EP-A-0 353 393 erwähnt. Das Einschreiben von Informationen erfolgt hierbei mit einer durch Laserstrahlung verursachten Veränderung der Absorption bzw. Reflektion in der speicheraktiven Aufzeichnungsschicht. Um im nahen IR-Bereich (NIR-Bereich) die einfachen Diodenlaser anwenden zu können, enthält die speicheraktive Schicht IR-Strahlung absorbierende Farbstoffe, zum Beispiel Cyaninfarbstoffe. Der nach der Bestrahlung erhaltene Informationspunkt weist daher eine niedrigere Absorption bzw. erhöhte Reflektion auf, die optisch ausgelesen werden kann.

Es wurden nun überraschend neue irreversibel thermochrome Verbindungen gefunden, die im NIR-Bereich keine Absorptionsbanden aufweisen, und sich bei Einwirkung von Wärme bzw. Wärmestrahlung in Produkte umwandeln, die im NIR-Bereich starke Absorptionsbanden besitzen. Die Verbindungen eignen sich daher hervorragend für speicheraktive Schichten in laseroptischen Aufzeichnungs- und Ausleseverfahren, insbesondere unter Verwendung von Diodenlasern und Laserstrahlung im NIR-Bereich, wobei zum Auslesen der eingeschriebenen Informationen die erhöhte Absorption bzw. erniedrigte Reflexion genutzt wird.

Ein Gegenstand der vorliegenden Erfindung sind Verbindungen der Formel I 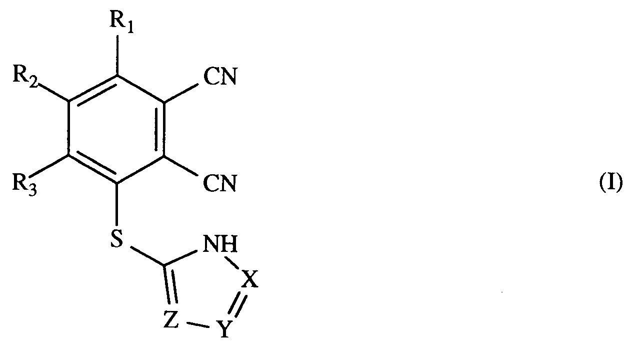
in welcher R₁, R₂ und R₃ unabhängig voneinander je Wasserstoff, eine geradkettige oder verzweigte C₁-C₁₂-Alkylgruppe, die unsubstituiert oder mit Alkoxy, Alkylthio, Aryloxy, Arylthio, Halogen, -CN, -COOH, -COOR₇, -NR₈R₉ oder -CONR₈R₉ substituiert ist, eine C₁-C₁₂-Alkoxygruppe, in der die Alkylgruppe geradkettig oder verzweigt und unsubstituiert oder mit Alkoxy, Alkylthio, Aryloxy, Arylthio, Halogen, -CN, -COOH, -COOR₇, -NR₈R₉ oder -CONR₈R₉ substituiert ist, eine C₁-C₁₂-Alkylthiogruppe, in der die Alkylgruppe geradkettig oder verzweigt und unsubstituiert oder mit Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkoxygruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkylthiogruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₆-C₁₀-Arylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₆-C₁₀-Aryloxygruppe, in der die Arylgruppe unsubstituiert oder mit Alkyl, Alkoxy, Alkylthio, Phenyl, Phenoxy, Phenylthio, Halogen, -CN, -COOH, -COOR₇, -NR₈R₉ oder -CONR₈R₉ substituiert ist, eine C₆-C₁₀-Arylthiogruppe, in der die Arylgruppe unsubstituiert oder mit Alkyl, Alkoxy, Alkylthio, Phenyl, Phenoxy, Phenylthio, Halogen, -CN, -COOH, -COOR₇, -NR₈R₉ oder -CONR₈R₉ substituiert ist, Nitro, Halogen, Cyano, -COOH, -COOR₇, -NR₈R₉ oder -CONR₈R₉ bedeuten, wobei R₇ C₁-C₂₀-Alkyl, C₅-C₈-Cycloalkyl, Phenyl oder Phenyl-C₁-C₄-alkyl bedeutet, und R₈ und R₉ unabhängig voneinander je Wasserstoff, C₁-C₈-Alkyl, Phenyl oder Phenyl-C₁-C₄-alkyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, eine geradkettige oder verzweigte C₁-C₁₂-Alkylgruppe, die unsubstituiert oder mit Alkoxy, Alkylthio, Aryloxy, Arylthio, Halogen, -CN, -COOH, -COOR₇, -NR₈R₉ oder -CONR₈R₉ substituiert ist oder eine C₆-C₁₀-Arylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen eine Gruppe 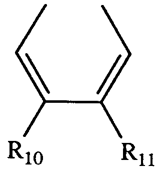
oder eine Tetramethylengruppe bilden, wobei R₁₀ und R₁₁ unabhängig voneinander Wasserstoff, C₁-C₆-Alkyl, Phenyl oder Phenyl-C₁-C₄-alkyl darstellen.

Typische geradkettige oder verzweigte C₁-C₁₂-Alkylgruppen, die als Reste R₁, R₂ und R₃ oder in den Resten R₁, R₂ und R₃ als Alkoxy- oder Alkylthiogruppen auftreten können, sind Methyl, Ethyl, n-Propyl, Isopropyl, n-Butyl, Isobutyl, sec. Butyl, tert. Butyl, n-Pentyl, 2-Pentyl, n-Hexyl, n-Heptyl, n-Octyl, Isooctyl, Nonyl, Decyl, Undecyl und Dodecyl. Innerhalb der Bedeutung dieser als bzw.in den Resten R₁, R₂ und R₃ vorliegenden geradkettigen oder verzweigten C₁-C₁₂-Alkylgruppen sind geradkettige oder verzweigte C₁-C₈-Alkylgruppen und inbesondere C₁-C₄-Alkylgruppen bevorzugt.

In den Resten R₁, R₂ und R₃ als C₅-C₈-Cycloalkyl, C₅-C₈-Cycloalkoxy und C₅-C₈-Cycloalkylthiogruppen bedeutet Cycloalkyl vorzugsweise Cyclohexyl.

In den Resten R₁, R₂ und R₃ als C₆-C₁₀-Aryl-, C₆-C₁₀-Aryloxy- und C₆-C₁₀-Arylthiogruppen bedeutet C₆-C₁₀-Aryl insbesondere Phenyl oder Naphthyl und besonders bevorzugt Phenyl.

Die Reste R₁, R₂ und R₃ können als C₁-C₁₂-Alkyl-, C₁-C₁₂-Alkoxy- und C₁-C₁₂-Aklythiogruppen ein- bis dreifach, bevorzugt einfach mit Alkoxy, Alkylthio, Aryl, Aryloxy, Arylthio, Halogen, -CN, -COOH, -COOR₇, -NR₈R₉ oder -CONR₈R₉ substituiert sein. Dabei können die in den Alkoxy- und Alkythiogruppen vorliegenden Alkylgruppen 1-12 Kohlenstoffatome enthalten. Vorzugsweise enthalten diese Alkylgruppen jedoch 1-6 Kohlenstoffatome und besonders bevorzugt 1-4 Kohlenstoffatome. In den Aryl-, Aryloxy- und Arylthiogruppen kann Aryl 6-10 Kohlenstoffatome enthalten und beispielsweise Phenyl oder Naphthyl bedeuten. Vorzugsweise bedeutet Aryl in diesen Gruppen Phenyl. Halogen bedeutet Fluor, Chlor, Brom oder Jod, insbesondere Fluor, Chlor oder Brom. Die in den Gruppen -COOR₇, -NR₈R₉ und -CONR₈R₉ vorkommenden Reste R₇, R₈ und R₉ bedeuten bevorzugt C₁-C₈-Alkyl, Phenyl oder Phenyl-C₁-C₄-alkyl, wobei Phenyl und der in der Phenyl-C₁-C₄-alkylgruppe vorliegende Phenylrest durch übliche Substituenten, wie zum Beispiel C₁-C₄-Alkyl, C₁-C₄-Alkoxy, Halogen oder Nitro substituiert sein können.

Die Reste R₁, R₂ und R₃ als C₅-C₈-Cycloalkyl-, C₅-C₈-Cycloalkoxy-, C₅-C₈-Cycloalkylthio-, C₆-C₁₀-Aryl-, C₆-C₁₀-Aryloxy- und C₆-C₁₀-Arylthiogruppen können ein- bis dreifach, bevorzugt einfach mit Alkyl, Alkoxy, Alkylthio, Aryl, Aryloxy, Arylthio, Halogen, -CN, -COOH, -COOR₇, -NR₈R₉ oder -CONR₈R₉ substituiert sein. Dabei können die Alkyl- Alkoxy- und Alkylthiogruppen 1-12 Kohlenstoffatome enthalten. Vorzugsweise enthalten diese Alkyl-, Alkoxy- und Alkylthiogruppen jedoch 1-8 Kohlenstoffatome und besonders bevorzugt 1-4 Kohlenstoffatome. In den Aryl-, Aryloxy- und Arylthiogruppen kann Aryl 6-10 Kohlenstoffatome enthalten und beispielsweise Phenyl oder Naphthyl bedeuten. Vorzugsweise bedeutet Aryl in diesen Gruppen Phenyl. Halogen bedeutet bevorzugt Fluor, Chlor oder Brom, insbesondere bevorzugt Fluor oder Chlor. Die in den Gruppen -COOR₇, -NR₈R₉ und -CONR₈R₉ vorkommenden Reste R₇, R₈ und R₉ bedeuten bevorzugt C₁-C₈-Alkyl, Phenyl oder Phenyl-C₁-C₄-alkyl, wobei Phenyl und der in der Phenyl-C₁-C₄-alkylgruppe vorliegende Phenylrest mit üblichen Substituenten, wie C₁-C₄-Alkyl, C₁-C₄-Alkoxy, Halogen oder Nitro substituiert sein können.

Die Reste R₁, R₂ und R₃ bedeuten bevorzugt unabhängig voneinander je Wasserstoff, eine C₁-C₈-Alkylgruppe, die unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₈-Alkoxygruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₈-Alkylthiogruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyl- oder Naphthylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyloxy- oder Naphthyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenylthio- oder Naphthylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, Fluor, Chlor, Brom, Nitro, Cyano, -COOH, -COOR₆, -NR₇R₈ oder -CONR₇R₈ bedeuten, wobei R₇ C₁-C₈-Alkyl oder Phenyl bedeutet und R₈ und R₉ unabhängig voneinander je Wasserstoff, C₁-C₈-Alkyl, Phenyl oder Phenyl-C₁-C₄-alkyl darstellen.

Besonders bevorzugt bedeuten die Reste R₁, R₂ und R₃ unabhängig voneinander je Wasserstoff, eine C₁-C₄-Alkylgruppe, die unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₄-Alkoxygruppe, in der die Alkylgruppe geradkettig oder verzweigt und unsubstituiert mit durch C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₄-Alkylthiogruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyl-oder Naphthylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyloxy- oder Naphthyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenylthio- oder Naphthylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, Fluor, Chlor, Brom, Nitro, Cyano, -COOH, -COOR₆, -NR₇R₈ oder -CONR₇R₈ bedeuten, wobei R₇ C₁-C₄-Alkyl oder Phenyl bedeutet und R₈ und R₉ unabhängig voneinander je Wasserstoff, C₁-C₄-Alkyl, Phenyl oder Benzyl darstellen.

Bei der in den Verbindungen der Formel I enthaltenen Azolylgruppe der Formel 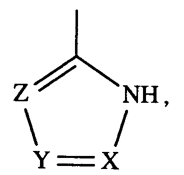
wobei X Stickstoff oder CR₄, Y Stickstoff oder CR₅ und Z Stickstoff oder CR₆ bedeuten und R₄, R₅ und R₆ die im Anspruch 1 angegebene Bedeutung haben, kann es sich um unsubstituiertes oder mit R₄, R₅, R₆ substituiertes Pyrrol-2-yl-, Imidazol-2-yl-, Imidazol-4-yl-, Imidazol-5-yl- Pyrazol-3-yl-, Pyrazol-5-yl-, 1H-1,2,3-Triazol-5-yl-, 2H-1,2,3-Triazol-5-yl-, 3H-1,2,3-Triazol-5-yl-, 1H-1,2,4-Triazol-5-yl-, 2H-1,2,4-Triazol-2-yl-, 4H-1,2,4-Triazol-2-yl-, 1H-1,2,3,4-Tetrazol-5-yl-, 2H-1,2,3,4-Tetrazol-5-yl-, Indol-2-yl-, Isoindol-2-yl-, Benzimidazol-2-yl-, Benzpyrazol-3-yl-, 4,5,6,7 Tetarhydro-Indol-2-yl-, 4,5,6,7 Tetrahydro-Isoindol-2-yl-, 4,5,6,7 Tetrahydro-Benzimidazol-2-yl-, 4,5,6,7 Tetrahydro-Benzpyrazol-3-yl- handeln, wobei die annellierten Benzolringe oder Tetrahydrobenzolringe durch die Reste R₁₀ und R₁₁ der in Anspruch 1 unter Formel I angegebenen Bedeutung substituiert sein können.

Die Reste R₄, R₅ und R₆ der in den nicht benzannellierten Azolresten vorliegenden Gruppen CR₄, CR₅ und CR₆ können unabhängig voneinander eine geradkettige oder verzweigte C₁-C₁₂-Alkylgruppe, die unsubstituiert oder mit Alkoxy, Alkylthio,Aryloxy, Arylthio, Halogen, -CN, -COOH, -COOR₇, -NR₈R₉ oder -CONR₈R₉ substituiert ist oder eine C₆-C₁₀-Arylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, bedeuten. Wenn X CR₄, Y CR₅ und Z CR₆ bedeuten sind bevorzugt R₄, R₅ und R₆ unabhängig voneinander C₁-C₄-Alkyl, C₁-C₄-Halogenalkyl oder Phenyl, wobei die Halogenalkylgruppe ein oder mehrere Halogenatome enthalten kann und unter Halogen insbesondere Fluor, Chlor oder Brom und vorzugsweise Fluor oder Chlor zu verstehen ist. Eine bevorzugte Halogenalkylgruppe ist Trifluormethyl. Die Reste R₁₀ und R₁₁ der gemeinsam von R₄ und R₅ bzw, R₅ und R₆ gebildeten Gruppe 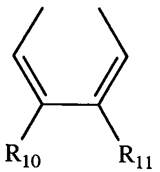
bedeuten vorzugsweise Wasserstoff, sodass die von den Resten R₄ und R₅ bzw. R₅ und R₆ gemeinsam gebildete Gruppe vorzugsweise eine 1,3- Butadienylengruppe ist.

Bevorzugte Azolylreste sind der Imidazol-2-ylrest und der 1,2,4-Triazol-3-ylrest.

Eine weitere bevorzugte Gruppe von Verbindungen der Formel I sind diejenigen, in welchen R₄, R₅ und R₆ unabhängig voneinander je C₁-C₄-Alkyl-, C₁-C₄-Halogenalkyl oder Phenyl bedeuten oder R₄ und R₅ oder R₅ und R₆ jeweils zusammen eine 1,3,-Butadienylengruppe darstellen, und R₁, R₂ und R₃ die oben unter Formel I genannten Bedeutungen haben, einschliesslich der im Anschluss an diese Bedeutungen genannten Bevorzugungen.

Ferner sind Verbindungen der Formel I bevorzugt, in welchen X CR₄, Y CR₅ und Z Stickstoff bedeutet, wobei R₄ und R₅, sowie R₁, R₂ und R₃ die oben unter Formel I angegebenen Bedeutungen haben, unter Einschluss der bevorzugten Bedeutungen, die im Anschluss an diese Angaben erläutert wurden. Von diesen Verbindungen sind wiederum diejenigen besonders bevorzugt, in welchen R₄ und R₅ unabhängig voneinander je Wasserstoff, C₁-C₄-Alkyl, C₁-C₄-Halogenalkyl oder Phenyl bedeuten oder R₄ und R₅ zusammen einen 1,3-Butadienylenrest darstellen, und R₁, R₂ und R₃ die oben unter Formel I angegebenen Bedeutungen haben, einschliesslich der im Anschluss an diese Bedeutungen genannten Bevorzugungen.

Ferner sind Verbindungen der Formel I bevorzugt, in welchen X CR₄, Y Stickstoff und Z CR₆ bedeutet, wobei R₄ und R₆, sowie R₁, R₂ und R₃ die oben unter Formel I angegebenen Bedeutungen haben, unter Einschluss der bevorzugten Bedeutungen, die im Anschluss an diese Angaben erläutert wurden. Von diesen Verbindungen sind wiederum diejenigen besonders bevorzugt, in welchen R₄ und R₆ unabhängig voneinander je Wasserstoff, C₁-C₄-Alkyl, C₁-C₄-Halogenalkyl oder Phenyl bedeuten.

Ebenfalls bevorzugt sind Verbindungen der Formel I in welchen X Stickstoff, Y CR₅ und Z CR₆ bedeutet, wobei R₅ und R₆, sowie R₁, R₂ und R₃ die oben unter Formel I angegebenen Bedeutungen haben, unter Einschluss der bevorzugten Bedeutungen, die im Anschluss an diese Angaben erläutert wurden. Von diesen Verbindungen sind wiederum diejenigen besonders bevorzugt, in welchen R₅ und R₆ unabhängig voneinander je Wasserstoff, C₁-C₄-Alkyl, C₁-C₄-Halogenalkyl oder Phenyl bedeuten oder R₅ und R₆ zusammen einen 1,3-Butadienylenrest darstellen, und R₁, R₂ und R₃ die oben unter Formel I angegebenen Bedeutungen haben, einschliesslich der im Anschluss an diese Bedeutungen genannten Bevorzugungen.

Weitere bevorzugte Verbindungen der Formel I sind diejenigen, in welchen X und Y Stickstoff bedeuten und Z CR₆ ist, wobei R₆, sowie R₁, R₂ und R₃ die oben unter Formel I angegebenen Bedeutungen haben, unter Einschluss der bevorzugten Bedeutungen, die im Anschluss an diese Angaben erläutert wurden. Von diesen Verbindungen sind wiederum diejenigen besonders bevorzugt, in welchen R₆ Wasserstoff, C₁-C₄-Alkyl, C₁-C₄-Halogenalkyl oder Phenyl bedeutet, und R₁, R₂ und R₃ die oben unter Formel I angegebenen Bedeutungen haben, einschliesslich der im Anschluss an diese Bedeutungen genannten Bevorzugungen.

Weiterhin sind Verbindungen der Formel I bevorzugt, in welchen X, Y und Z Stickstoff bedeuten, wobei R₁, R₂ und R₃ die oben unter Formel I angegebenen Bedeutungen haben, unter Einschluss der bevorzugten Bedeutungen, die im Anschluss an diese Angaben erläutert wurden.

Wie oben ausgeführt wurde, können die Reste R₁, R₂ und R₃ unabhängig voneinander die oben näher erläuterten Bedeutungen haben. Gemäss einer besonderen Ausführungsform der vorliegenden Erfindung sind solche Verbindungen der Formel I bevorzugt, in welchen R₁ und R₃ Wasserstoff bedeuten und R₂, X, Y und Z die unter der allgemeinen Formel I angegebene Bedeutung haben.

Für eine weitere bevorzugte Untergruppe von Verbindungen der allgemeinen Formel I bedeuten R₁ und R₃ Wasserstoff und R₂ C₁-C₈-Alkyl, C₁-C₈-Alkoxy, C₁-C₈-Alkylthio, Cyclohexyl, Cyclohexyloxy, Cyclohexylthio, Phenyl, Phenoxy, Phenylthio, Fluor, Chlor, Brom, Nitro, Cyano, -COOH, -COOR₇, -NR₈R₉ oder -CONR₈R₉, wobei R₇ C₁-C₈-Alkyl, Phenyl-C₁-C₄-alkyl oder Phenyl bedeutet, und R₈ und R₉ unabhängig voneinander je Wasserstoff, C₁-C₈-Alkyl, Phenyl oder Phenyl-C₁-C₄-alkyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, C₁-C₄-Alkyl, C₁-C₄-Halogenalkyl oder Phenyl bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen auch eine 1,3-Butadienylengruppe darstellen können.

Für eine besonders bevorzugte Untergruppe von Verbindungen der allgemeinen Formel I bedeuten R₁ und R₃ Wasserstoff und R₂ C₁-C₄-Alkyl, C₁-C₄-Alkoxy, C₁-C₄-Alkylthio, Cyclohexyl, Cyclohexyloxy, Cyclohexythio, Phenyl, Phenoxy, Phenylthio, Fluor, Chlor, Brom, Nitro, Cyano, -COOH, -COOR₇, -NR₈R₉ oder -CONR₈R₉, wobei R₇ C₁-C₄-Alkyl, Phenyl oder Benzyl bedeutet, R₈ und R₉ unabhängig voneinander je Wasserstoff, C₁-C₄-Alkyl, Phenyl oder Benzyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, C₁-C₄-Alkyl, C₁-C₄-Halogenalkyl oder Phenyl bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen auch eine 1,3-Butadienylengruppe darstellen können.

Besonders bevorzugt sind Verbindungen der Formel I, in welchen R₁ und R₃ Wasserstoff bedeuten, R₂ für Wasserstoff, Nitro, C₁-C₄-Alkylthio oder Phenylthio steht, wobei die Phenylgruppe durch C₁-C₄-Alkyl, C₁-C₄-Alkoxy oder C₁-C₄-Alkylthio substituiert sein kann, X CR₄, Y CR₅ oder Stickstoff und Z Stickstoff bedeutet, wobei R₄ und R₅ unabhängig voneinander Wasserstoff, C₁-C₄-Alkyl, C₁-C₄-Halogenalkyl, Phenyl oder zusammen eine Gruppe 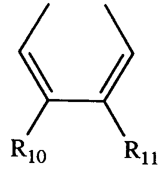
darstellen, wobei R₁₀ und R₁₁ unabhängig voneinander Wasserstoff oder C₁-C₄-Alkyl bedeuten.

Ein weiterer Gegenstand der vorliegenden Erfindung ist ein Verfahren zur Herstellung der Verbindungen der Formel I. Nach diesem Verfahren werden die Verbindungen der allgemeinen Formel I hergestellt, indem man ein substituiertes Phthalsäuredinitril der Formel II 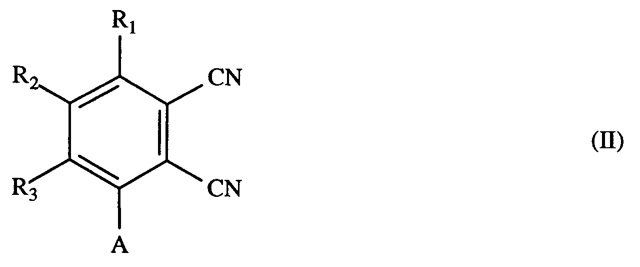
in welcher R₁, R₂ und R₃ die unter Formel I angegebene Bedeutung haben und A für Halogen oder eine Nitrogruppe steht, in einem inerten Lösungsmittel in Gegenwart einer Base mit einem Azol der allgemeinen Formel III 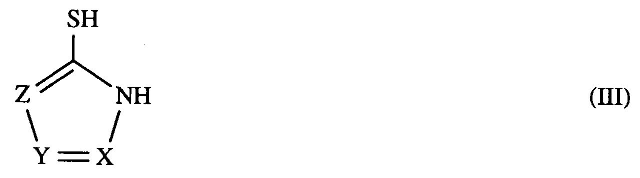
in welcher X, Y und Z die unter Formel I angegebene Bedeutung haben, umsetzt.

Die Ausgangsmaterialien der Formeln II und III sind teils bekannte und teils neue Verbindungen. Die neuen Verbindungen der Formeln II oder III können in analoger Weise wie die entsprechenden, bereits bekannten Verbindungen hergestellt werden.

Von den Ausgangsmaterialien der Formel II sind diejenigen bevorzugt, in welchen A eine Nitrogruppe ist.

Als inerte Lösungsmittel können aliphatische und aromatische Kohlenwasserstoffe, wie Hexan, Cyclohexan, Toluol, Xylol und Mesitylen, aliphatische und aromatische Halogenkohlenwassersroffe, wie Methylenchlorid, Chloroform, Kohlenstofftetrachlorid, Trichlorethan, Tetrachlorethan, Trichlorethylen, Chlorbenzol und Dichlorbenzol, Ether, wie Diethylether, Dibutylether, Diisobutylether, Tetrahydrofuran und Dioxan, ferner Dimethylsulfoxid und Säureamidderivate, wie N,N-Dimethylformamid, N,N-Dimethylacetamid und N-Methyl-2-pyrrolidon, Nitrile, wie Acetonitril, Ketone, wie Aceton und Methylethylketon, Carbonsäureester, wie Ethylacetat, verwendet werden. Bevorzugte Lösungsmittel sind Dimethylsulfoxid, N,N-Dimethylformamid, N-Methyl-2-pyrrolidon, Tetrahydrofuran und Dioxan.

Als Basen kommen vor allem Alkalimetall- und Erdalkalimetallhydroxide, -carbonate und -hydrogencarbonate, wie Natriumhydroxid, Kaliumhydroxid, Natriumcarbonat, Natriumhydrogencarbonat, Kaliumhydroxid, Kaliumcarbonat, Kaliumhydrogencarbonat, Calciumhydroxid, Calciumcarbonat und Calciumhydrogencarbonat, und ferner tertiäre Amine, wie Triethylamin, Pyridin und Chinolin in Betracht. Bevorzugte Basen sind Kaliumcarbonat, Triethylamin und Pyridin. Eine besonders bevorzugte Base ist Kaliumcarbonat.

Die Umsetzung kann bei Raumtemperatur oder darunter oder bei erhöhter Temperatur durchgeführt werden. Geeignete Reaktionstemperaturen liegen im Bereich von 0°C bis 100°C. Vorzugsweise wird die Umsetzung bei einer Temperatur im Bereich von 0°C bis 60°C durchgeführt.

Ein weiterer Gegenstand der vorliegenden Erfindung sind Verbindungen der allgemeinen Formel IV 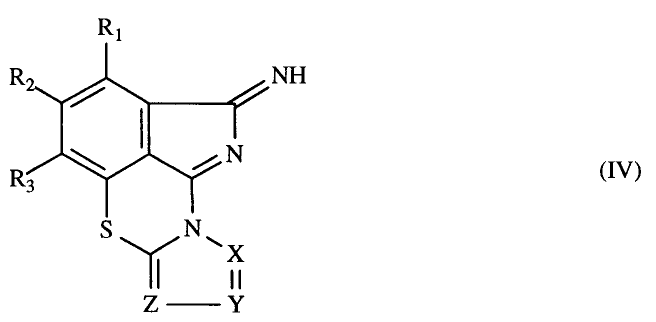
in welcher R₁, R₂, R₃, X, Y und Z die unter Formel I angegebene Bedeutung haben.

Die bevorzugten Bedeutungen von R₁, R₂, R₃, X, Y und Z entsprechen denjenigen, die unter Formel I für diese Reste angegeben wurden.

Die Verbindungen der allgemeinen Formel IV können gemäss vorliegender Erfindung hergestellt werden, indem man eine Verbindung der Formel I auf eine Temperatur im Bereich von 75°C bis 300°C, vorzugsweise 90°C bis 160°C, erhitzt.

Das Erhitzen kann in Abwesenheit oder in Anwesenheit eines inerten Lösungsmittels durchgeführt werden. Geeignete Lösungsmittel sind beispielsweise aliphatische und aromatische Kohlenwasserstoffe, wie Hexan, Heptan, Oktan, Toluol, Xylol, Mesitylen, Tetrahydronaphthalin und Decahydronaphthalin, sowie aliphatische und aromatische Halogenkohlenwasserstoffe, wie Trichlorethan, Tetrachlorethan, Trichlorethylen, Chlorbenzol und Dichlorbenzol. Bevorzugte Lösungsmittel sind Toluol, Xylol, Mesitylen und o-Dichlorbenzol.

Falls das Verfahren zur Herstellung einer Verbindung der allgemeinen Formel IV in einem Lösungsmittel durchgeführt wird, scheiden sich die Verbindungen der Formel IV in der Regel in fester Form aus der Reaktionslösung ab und können in einfacher Weise durch Filtration abgetrennt werden. Falls ohne Lösungsmittel gearbeitet wird, ist es vorteilhaft, das Erhitzen der Verbindung der Formel I im Vakuum vorzunehmen und die gebildete Verbindung der Formel IV durch Sublimation aus dem Reaktionsgemisch abzutrennen.

Die Erhitzungsdauer kann je nach Verbindung der Formel I und der angewandten Temperatur variieren und liegt in der Regel im Bereich von 1 Minute bis 10 Stunden und bevorzugt im Bereich von 1 Stunde bis 5 Stunden.

Die Verbindungen der Formeln I und IV sind kristallin, farblos bis hellgelb gefärbt und in organischen Lösungsmitteln und in Polymeren löslich. Ferner sind die Verbindungen der Formeln I und IV irreversibel thermochrom, d. h. sie zeigen bei der Einwirkung von Wärme bzw. Wärmestrahlung eine ausgeprägte Farbänderung, die von farblos bis hellgelb über grün, braun bis zu schwarz verläuft. Durch die Einwirkung von Wärme bzw. Wärmestrahlung wird die Lichtabsorption der Verbindungen der Formeln I und IV nach längeren Wellenlängen hin verschoben. Die Umwandlungsgeschwindigkeit ist überraschend hoch und kann je nach Menge und Dicke der Probe und der Intensität der thermischen Strahlung innerhalb einer Zeitspanne von weniger als 3 Sekunden erfolgen. Die thermochemische Umwandlung ist irreversibel.

Die Verbindungen der Formeln I und IV eignen sich daher ausgezeichnet als wärmeempfindlicher Aktivbestandteil für verschiedene Verfahren zur Informationsaufzeichnung unter Verwendung von Wärme bzw. Wärmestrahlung, oder als Aktivbestandteil in thermochromen Anzeigeelementen.

Ein weiterer Gegenstand der Erfindung ist ein Material zur optischen Aufzeichnung und zur Speicherung von Informationen, bei dem auf einem Träger mindestens eine Schicht mindestens eine der Verbindungen der Formeln I und IV ohne Bindemittel oder zusammen mit einem Bindemittel als speicheraktive Schicht aufgebracht ist. Der Träger ist bevorzugt transparent.

Geeignete Träger sind zum Beispiel Metalle, Metallegierungen, Gläser, Mineralien, Keramiken, Papier und duroplastische oder thermoplastische Kunststoffe. Der Träger kann eine Dicke von 0,01 mm bis 1 cm, bevorzugt 0,1 mm bis 0,5 cm aufweisen. Bevorzugte Träger sind Gläser und homo- oder copolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze.

Die Kunststoffträger können nach den für duroplastische und thermoplastische üblichen Misch- und Formgebungsverfahren wie zum Beispiel Giess-, Press-, Spritzguss- und Extrusionsverfahren hergestellt werden.

Auf dem Träger können ein oder mehrere, zum Beispiel 1 bis 10, bevorzugt 1 bis 5 und besonders bevorzugt 1 bis 3 Schichten aus gleichen oder verschiedenen Verbindungen der Formel I aufgebracht sein. Die Anzahl der Schichten und weiterer Schichten richtet sich hauptsächlich nach der optischen Dichte des Schichtaufbaus, die bei der zur Aufzeichnung verwendeten Wellenlänge noch eine ausreichende Transmission gewährleisten muss.

Die Schichtdicke der Schicht aus Verbindungen der Formeln I und/oder IV beträgt zum Beispiel 100 bis 3000 Å, bevorzugt 100 bis 2000 Å und besonders bevorzugt 200 bis 1500 Å. Sofern ein Bindemittel mitverwendet wird, kann die Schichtdicke 0,1 bis 500 µm, bevorzugt 1 bis 200 µm und besonders bevorzugt 1 bis 100 µm betragen.

Auf der speicheraktiven Schicht oder auf dem Träger kann eine Reflexionsschicht aufgebracht sein, die eine Dicke von zum Beispiel 100 bis 5000 Å, bevorzugt 100 bis 3000 Å und besonders von 300 bis 1500 Å aufweisen kann. Als reflektierendes Material eignen sich besonders Metalle, die die zur Aufzeichnung und Wiedergabe verwendete Laserstrahlung gut reflektieren. Besonders bevorzugt ist aus Gründen der hohen Reflektivität und leichten Herstellbarkeit eine Reflexionsschicht aus Aluminium oder Gold.

Die je nach Schichtaufbau oberste Schicht, zum Beispiel die Reflexionsschicht, die speicheraktive Schicht oder eine weitere Hilfsschicht wird zweckmässig mit einer Schutzschicht versehen, die eine Dicke von 0,1 bis 100 µm, bevorzugt 0,1 bis 50 µm und besonders bevorzugt 0,5 bis 15 µm aufweisen kann. Als Schutzmaterial eignen sich hauptsächlich Kunststoffe, die in dünner Schicht entweder direkt oder mit Hilfe von Haftschichten auf den Träger oder die oberste Schicht aufgetragen sind. Man wählt zweckmässig mechanisch und thermisch stabile Kunststoffe mit guten Oberflächeneigenschaften, die noch modifiziert, zum Beispiel beschrieben werden können. Es kann sich sowohl um duroplastische wie auch thermoplastische Kunststoffe handeln. Bevorzugt sind strahlungsgehärtete (zum Beispiel mit UV-Strahlung) Schutzschichten, die besonders einfach und wirtschaftlich herstellbar sind. Strahlungshärtbare Materialien sind in grosser Vielzahl bekannt. Beispiele für strahlungshärtbare Monomere und Oligomere sind Acrylate und Methacrylate von Diolen, Triolen und Tetrolen, Polyimide aus aromatischen Tetracarbonsäuren und aromatischen Diaminen mit C₁-C₄-Alkylgruppen in mindestens zwei Orthostellungen der Aminogruppen, und Oligomere mit Dialkyl-, zum Beispiel Dimethylmaleinimidylgruppen. Spezifische Beispiele sind UV-vernetzbare Polymere auf der Basis von Polyacrylaten wie zum Beispiel RENGOLUX® RZ 3200/003 oder 3203/001 der Firma Morton International-Dr. Renger.

Die speicheraktive Schicht kann als gleichmässige Mischung von Verbindungen der Formeln I und/oder IV mit einem transparenten Bindemittel, zum Beispiel einem Kunststoff vorliegen. Die Schichtdicke der Mischung mit Bindemittel (appliziert z.B. mit einer Belackungsanlage) kann zum Beispiel 0,1 bis 100 µm, bevorzugt 0,5 bis 50 µm und besonders bevorzugt 0,5 bis 5 µm betragen. Die Mischung mit dem Bindemittel kann 0,1 bis 95 Gew.-%, bevorzugt 1 bis 80 Gew.-% und besonders bevorzugt 1 bis 60 Gew.-% Verbindungen der Formeln I und/oder IV enthalten, bezogen auf die Gesamtmenge an Bindemittel und Lösungsmittel. Als transparente Bindemittel eignen sich zum Beispiel die zuvor für das Substrat erwähnten Kunststoffe. Bevorzugt sind insbesondere Polycarbonate, sowie ferner Polymethacrylate, duroplastische Polyester und Epoxidharze. Bei der Mischung kann es sich auch um den Träger selbst handeln, z.B. um ein Polycarbonat, dem Verbindungen der Formeln I und/oder IV einverleibt sind.

Die erfindungsgemäss zu verwendenden Aufzeichnungsmaterialien können nach an sich bekannten Verfahren hergestellt werden, wobei je nach verwendeten Materialien und deren Funktionsweise unterschiedliche Beschichtungsmethoden angewendet werden können.

Geeignete Beschichtungsverfahren sind zum Beispiel Tauchen, Giessen, Streichen, Rakeln, Schleudergiessen und Aufdampfverfahren, die im Hochvakuum durchgeführt werden. Bei der Anwendung von zum Beispiel Giessverfahren werden im allgemeinen Lösungen in organischen Lösungsmitteln verwendet, die noch zusätzlich ein Bindemittel enthalten können. Bei der Verwendung von Lösungsmitteln ist darauf zu achten, dass die verwendeten Träger gegen diese Lösungsmittel unempfindlich sind. Bevorzugt werden alle Schichten mittels Aufdampfverfahren besonders im Vakuum hergestellt. Geeignete Beschichtungsverfahren sind zum Beispiel in der EP-A-0 401 791 beschrieben.

Das Aufbringen der Aufzeichnungsschicht(en) und der metallischen Reflexionsschichten erfolgt bevorzugt durch Aufdampfen im Vakuum. Das aufzubringende Material wird zunächst in ein geeignetes Gefäss gefüllt, das gegebenenfalls mit einer Widerstandsheizung versehen ist, und dann in eine Vakuumkammer gesetzt. Der zu bedampfende Träger wird oberhalb des Gefässes mit dem aufzudampfenden Material in einen Halter eingesetzt. Dieser ist so konstruiert, dass der Träger gegebenenfalls rotiert (z.B. mit 10 Upm) und geheizt werden kann. Die Vakuumkammer wird auf etwa 1,3 · 10⁻⁵ bis 1,3 · 10⁻⁶ mbar (10⁻⁵ bis 10⁻⁶ Torr) evakuiert, und die Heizung wird so eingestellt, dass die Temperatur des aufzudampfenden Materials bis zu dessen Verdampfungstemperatur ansteigt. Die Verdampfung wird so lange fortgesetzt, bis die aufgedampfte Schicht die gewünschte Dicke hat. Je nach Systemaufbau wird zuerst die organische Aufzeichnungsverbindung und dann die reflektierende Schicht aufgebracht, oder es kann umgekehrt verfahren werden. Auf das Aufbringen einer reflektierenden Schicht kann gegebenenfalls verzichtet werden.

Die Sputtertechnik wird besonders wegen der hohen Haftfähigkeit zum Träger für das Aufbringen der metallischen Reflexionsschicht bevorzugt. Das aufzubringende Material (z.B. Aluminium) in Form einer Platte wird als eine "Target"-Elektrode benutzt, während der Träger auf der Gegenelektrode befestigt wird. Zuerst wird die Vakuumkammer auf etwa 10⁻⁶ Torr evakuiert und dann inertes Gas, wie z.B. Argon, bis zu etwa 10⁻³ Torr eingeführt. Zwischen der "Target"-Elektrode und der Gegenelektrode wird eine hohe Gleich- oder Radiofrequenz-Spannung von mehreren kV, gegebenenfalls unter Verwendung von Permanentmagneten ("Magnetron-Sputtering"), angelegt, um Ar⁺-Plasma zu erzeugen. Die durch die Ar⁺-Ionen von der "Target"-Elektrode gesputterten (herausgeschlagenen) Metallteilchen werden auf dem Substrat gleichmässig und fest deponiert. Die Beschichtung erfolgt innerhalb von einigen 10 Sekunden bis mehreren Minuten je nach Targetmaterialien, Sputtermethode und Sputterbedingungen. Diese Sputtertechnik ist in Fachbüchern ausführlich beschrieben (wie z.B. W. Kern und L. Vossen, "Thin Film Processes", Academic Press, 1978).

Die Dicke der beim Aufdampfen gebildeten Schichten kann mit Hilfe bekannter Verfahren überwacht werden, z.B. mit Hilfe eines optischen Systems, welches während der Aufdampfung das Reflexionsvermögen der mit dem Absorptionsmaterial bedeckten Reflexionsoberfläche misst. Vorzugsweise wird das Wachsen der Schichtdicke mit einem Schwingquarz verfolgt.

Schutzschichten und Verfahren zu deren Aufbringen sind bekannt. Es handelt sich in der Regel um organische Polymere, die thermoplastisch oder vernetzt sein können. Bevorzugt werden strahlungsvernetzte Polymere, die sich aus strahlungsvernetzbaren Monomeren oder Oligomeren herstellen lasse verwendet. Beispiele für strahlungshärtbare Monomere und Oligomere sind Acrylate und Methacrylate von Diolen, Triolen und Tetrole. Die Schichten können durch Spritz-, Sprüh-, oder Giessverfahren hergestellt werden. Bevorzugt werden sie mittels Belackungsanlagen im Schleuderverfahren ("spin coater") aufgetragen.

Der Aufbau des erfindungsggemässen Aufzeichnungsmaterials richtet sich hauptsächlich nach der Auslesemethode; bekannte Funktionsprinzipien sind die Messung der Veränderung der Transmission oder der Reflexion. Wenn das Aufzeichnungsmaterial gemäss der Veränderung der Lichttransmission aufgebaut wird, kommt zum Beispiel folgende Struktur in Frage: Transparenter Träger/Aufzeichnungsschicht (gegebenenfalls mehrschichtig) und falls zweckmässig, transparente Schutzschicht. Das Licht zur Aufzeichnung sowie zum Auslesen kann entweder von der Trägerseite oder der Aufzeichnungsschicht bzw. gegebenenfalls der Schutzschichtseite eingestrahlt werden, wobei sich der Lichtdetektor immer auf der Gegenseite befindet.

Wenn das Aufzeichnungsmaterial gemäss der Veränderung der Reflexion aufgebaut wird, so können zum Beispiel folgende andere Strukturen zur Anwendung kommen: Transparenter Träger/Aufzeichnungsschicht (gegebenenfalls mehrschichig)/Reflexionsschicht und falls zweckmässig, Schutzschicht (nicht unbedingt transparent), oder Träger (nicht unbedingt transparent)/Reflexionsschicht/Aufzeichnungsschicht und falls zweckmässig transparente Schutzschicht. Im ersten Fall wird das Licht von der Trägerseite eingestrahlt, während im letzen Falls die Strahlung von der Aufzeichnungsschicht- bzw. gegebenenfalls von der Schutzschichtseite einfällt. In beiden Fällen befindet sich der Lichtdetektor auf gleicher Seite wie die Lichtquelle. Der ersterwähnte Aufbau des erfindungsgemäss zu verwendenden Aufzeichnungsmaterials ist im allgemeinen bevorzugt.

Das erfindungsgemäss zu verwendende Material eignet sich hervorragend zur Aufzeichnung von Informationen durch Bestrahlung mit Lasern im NIR-Bereich. Nach der Bestrahlung wird eine starke Zunahme der Absorption beobachtet. Die Veränderung der Absorption bzw. Transmission kann daher zum Auslesen der Information verwendet werden, wobei keine Zerstörung der eingeschriebenen Information durch die zum Auslesen verwendete Laserstrahlung stattfindet. Die Informationen können daher mehrfach ausgelesen werden.

Geeignete Laser sind zum Beispiel handelsübliche Diodenlaser, insbesondere Halbleiter-Diodenlaser, zum Beispiel GaAsAl-, InGaAlP- oder GaAs-Laser einer Wellenlänge von 780, 650 beziehungsweise 830 nm. Die Aufzeichnung kann mit Hilfe eines Lichtmodulators Punkt für Punkt vorgenommen werden.

Die zur Aufzeichnung verwendete Energie der Laserstrahlung kann zum Beispiel von 0,1 bis 10 nJ/Markierung (Bit), bevorzugt von 0,2 bis 5 nJ/Markierung (Bit) und besonders bevorzugt 0,8 bis 3 nJ/Markierung (Bit) betragen. Die Energiemenge wird im wesentlichen durch die Bestrahlungszeit gesteuert, zum Beispiel durch Pulse im Bereich von einigen 10 bis 100 Nanosekunden.

Das erfindungsgemässe Aufzeichnungsmaterial ermöglicht eine Speicherung von Informationen mit hoher Informationsdichte, Zuverlässigkeit und Beständigkeit, welche sich durch eine sehr gute mechanische und thermische Stabilität sowie durch eine hohe Lichtstabilität und scharfe Randzonen auszeichnen. Besonders vorteilhaft sind die hohe Lichtempfindlichkeit und das überraschend hohe Signal/Rauschverhältnis von Trägermaterial zur Informationsmarkierung, das ein einwandfreies Auslesen zulässt. Das optische Aufzeichnungssystem ist einfach und kostengünstig. Das Einschreiben der Information kann durch gerasterte, holographische oder photographische Belichtung der speicheraktiven Schicht erfolgen.

Das Auslesen der Information erfolgt durch Messung der Absorption nach dem Reflexionsverfahren oder Transmissionsverfahren unter Verwendung von Laserstrahlung, wobei es besonders vorteilhaft ist, dass Laserstrahlung der zur Aufzeichnung benutzten Wellenlänge verwendet werden kann, also auch kein zweiter Laser eingesetzt werden muss. In einer bevorzugten Ausführungsform erfolgt das Aufzeichnen und Auslesen der Information bei gleicher Wellenlänge. Beim Auslesen verwendet man im allgemeinen energiearme Laser, deren Strahlungsintensität gegenüber der zur Aufzeichnung verwendeten Laserstrahlung zum Beispiel um das zehn- bis fünfzigfache reduziert ist. Die Informationen können beim erfindungsgemäss verwendeten Aufzeichnungsmaterial ein- oder mehrfach ausgelesen werden. Die Veränderung im Absorptionsspektrum bzw. die gespeicherte Information kann mit einem Photodetektor unter Verwendung eines energiearmen Lasers abgelesen werden. Geeignete Photodetektoren umfassen PIN-Photodioden, welche es ermöglichen, die spektralen Veränderungen durch Transmission oder Absorption und insbesondere Reflexion zu messen.

Das erfindungsgemässe Material kann folgenden Aufbau aufweisen:
(a) transparenter Träger, (b) Aufzeichnungsschicht und (c) transparente Schutzschicht;
oder
(a) transparenter Träger, (b) Aufzeichnungsschicht, (c) Reflexionsschicht und (d) Schutzschicht;
oder
(a) Träger, (b) Reflexionsschicht, (c) Aufzeichnungsschicht und (d) transparente Schutzschicht.

Ein weiterer Gegenstand der Erfindung ist ein Verfahren zum bildhaften Aufzeichnen, bevorzugt optischen Aufzeichnen und Speichern von Informationen in Form von Bits, bei dem man ein erfindungsgemässes Aufzeichnungsmaterial bildmässig Wärme oder einer Wärmestrahlung, oder punktweise oder linienweise einer Laserstrahlung im NIR-Bereich aussetzt.

Ein anderer Gegenstand der Erfindung ist ein Informationen enthaltendes Material, bei dem in der Aufzeichnungsschicht eines erfindungsgemässen Aufzeichnungmaterials Bilder oder Markierungen in Form von Bits aufgebracht bzw. eingeschrieben sind, die im nahen Infrarotbereich gegenüber der unveränderten Umgebung eine verminderte Reflexion und erhöhte Absorption aufweisen.

Ein anderer Gegenstand der Erfindung ist eine thermochrome Zusammensetzung, die als thermochrome Komponente mindestens eine Verbindung der Formel I und/oder mindestens eine Verbindung der Formel IV enthält. Die thermochrome Komponente ist bevorzugt in einer Menge von 0,001 bis 20 Gew.% bezogen auf die Zusammensetzung enthalten.

Ein anderer Gegenstand der Erfindung ist eine thermochrome Zusammensetzung, enthaltend
a) ein farbloses organisches Lösungsmittel, ein Polymer oder ein organisches Glas, und
b) gelöst, eingemischt oder als Schicht auf mindestens einer Oberfläche mindestens eine Verbindung der Formel I und/oder mindestens eine Verbindung der Formel IV . Die Verbindungen Formeln I und IV sind bevorzugt in einer Menge von 0,001 bis 20 Gew.% bezogen auf die Komponente a) enthalten.

Ein weiterer Gegenstand der Erfindung ist die Verwendung von Verbindungen der Formeln I oder IV als irreversible thermochrome Systeme zur Kontrastbildung, Lichtabsorption als thermochrome Farbindikatoren.

Ein weiterer Gegenstand der Erfindung ist die Verwendung der Verbindung der Formel I oder IV zur irreversiblen optischen Speicherung von Information, wobei die Information in einer die Verbindung enthaltenden, speicheraktiven Schicht mit Wärmestrahlung, bevorzugt mit IR-Laserstrahlen, eingeschrieben wird.

Ein weiterer Gegenstand der Erfindung ist die Verwendung der Verbindung der Formel I oder IV als Aktivbestandteil in thermochromen Anzeigeelementen.

Ein weiterer Gegenstand der Erfindung ist die Verwendung der Verbindung der Formel I oder IV als thermochromer Aktivbestandteil für Bilderzeugungsverfahren unter Einwirkung von Wärme oder Wärmestrahlung.

Mit den erfindungsgemässen Verbindungen bzw. Materialien können CD-kompatible optische Aufzeichnungsmaterialien hergestellt werden, die als Computerspeicher, als photographische Speicher oder als Tonträger Verwendung finden können. Ferner können thermochrome Anzeigeelemente hergestellt werden sowie Thermophotographien und Thermodrucke.

Die vorliegende Erfindung wird durch die nachfolgenden Beispiele näher erläutert.

### A) Herstellungsbeispiele

### Beispiel A1: 3-(Imidazol-2-ylthio)-phthalsäuredinitril

5,79 g (57,8 mMol) 2-Mercaptoimidazol, 23,97 g (173,4 mMol) K₂CO₃ und 70 ml DMSO werden unter Rühren auf 15°C abgekühlt.Dann wird während 15 Minuten eine Lösung von 10,0 g (57,8 mMol) 3-Nitrophthalsäuredinitril in 30 ml DMSO zugetropft, wobei die Temperatur auf 15°C gehalten wird. Dann wird das Gemisch langsam auf 25°C erwärmt und 1 Stunde bei 25°C gerührt. Das Gemisch wird in eine Lösung von 18 ml Eisessig in 500 ml Wasser ausgetragen, wobei entstehende nitrose Gase mit Stickstoff ausgeblasen werden. Es wird 15 Minuten bei 10°C nachgerührt, der Niederschlag abfiltriert und zwei Mal mit Wasser gewaschen. Das Produkt wird in Tetrahydrofuran/Toluol gelöst, die Lösung über Na₂SO₄ getrocknet und eingedampft. Der Rückstand wird aus Dioxan umkristallisiert. Es werden 8,54 g (65% d. Theorie) reines 3-(Imidazol-2-ylthio)-phthalsäuredinitril erhalten.

| Elementaranalyse: C₁₁H₆N₄S (MG 226,26) | | | | |
|---|---|---|---|---|
| Ber.: | C 58,39 | H 2,67 | N 24,76 | S 14,17 |
| Gef.: | C 58,71 | H 2,76 | N 24,56 | S 13,81 |

Massenspektrum (m/e): 226 (M⁺, Basispeak).

Beim Erhitzen verfärbt sich die Substanz unter Gelb-, dann Grün- und schliesslich Schwarzfärbung.

In analoger Weise werden bei einer Reaktionstemperatur von 25°C die folgenden Verbindungen erhalten:

### Beispiel A2:

3-(4,5-Dimethylimidazol-2-ylthio)-phthalsäuredinitril (Reaktionszeit: 25 Minuten; MS: 254 (Basispeak); Ausbeute: 50% d. Theorie). Beim Erhitzen verfärbt sich die Substanz unter Gelb-, dann Grün- und schliesslich Schwarzfärbung.

### Beispiel A3:

3-(4-Phenylimidazol-2-ylthio)-phthalsäuredinitril (Reaktionszeit: 15 Minuten; MS: 302 (Basispeak) Ausbeute: 40% d. Theorie; Tautomerengemisch). Beim Erhitzen verfärbt sich die Substanz unter-Gelb-, dann Grün- und schliesslich Schwarzfärbung.

### Beispiel A4:

3-(4,5-Diphenylimidazol-2-ylthio)-phthalsäuredinitril (Reaktionszeit: 45 Minuten; MS: 378 (Basispeak); Ausbeute: 79% d. Theorie). Beim Erhitzen verfärbt sich die Substanz unter Gelb-, dann Grün- und schliesslich Schwarzfärbung.

### Beispiel A5:

3-(Benzimidazol-2-ylthio)-phthalsäuredinitril (Reaktionszeit: 80 Minuten; MS: 276 (Basispeak); Ausbeute: 24% d. Theorie). Beim Erhitzen verfärbt sich die Substanz unter Gelb-, dann Grün- und schliesslich Schwarzfärbung.

### Beispiel A6:

3-(5-Methylbenzimidazol-2-ylthio)-phthalsäuredinitril (Reaktionszeit: 60 Minuten; MS: 290 (Basispeak); Ausbeute: 20% d. Theorie; Tautomerengemisch). Beim Erhitzen verfärbt sich die Substanz unter Gelb-, dann Grün- und schliesslich Schwarzfärbung.

### Beispiel A7:

3-(1(H)-1,2,4-Triazol-3-ylthio)-phthalsäuredinitril (Reaktionszeit: 90 Minuten; MS: 227 (Basispeak); Ausbeute: 37% d. Theorie; es sind 3 Tautomere möglich; nach NMR-Spektrum 1 Hauptkomponente). Beim Erhitzen verfärbt sich die Substanz unter Gelb-, dann Grün- und schliesslich Schwarzfärbung.

### Beispiel A8:

3-(5-Trifluormethyl-1(H)-1,2,4-triazol-3-ylthio)-phthalsäuredinitril (Reaktionszeit: 60 Minuten; MS: 295 (Basispeak); Ausbeute: <10% d. Theorie; vermutlich Tautomerengemisch). Beim Erhitzen verfärbt sich die Substanz unter Gelb-, dann Grün- und schliesslich Schwarzfärbung.

### Beispiel A9: 3-(Imidazol- 2-ylthio)-5- nitrophthalsäuredinitril

0,92 g (9,17 mMol) 2-Mercaptoimidazol, 3,80 g (27,52 mMol) K₂CO₃ und 15 ml Tetrahydrofuran werden in einem Eis/Trockeneisbad auf -5°C abgekühlt. Dann wird der Suspension eine Lösung von 2,0 g (9,17 mMol) 3,5-Dinitrophthalsäuredinitril in 10 ml Tetrahydrofuran tropfenweise zugegeben. Anschliessend wird das Gemisch in eine Lösung von 3 ml Eisessig in 200 ml Wasser ausgetragen. Die entstandene Emulsion wird mit Tetrahydrofuran/Toluol extrahiert, die vereinigten Extrakte über Na₂SO₄ getrocknet und bei einer 60°C nicht überschreitenden Temperatur eingedampft. Der Rückstand wird aus Tetrahydrofuran/Toluol umkristallisiert. Es werden 1,43 g (57% d. Theorie) rohes 3-(Imidazol-2-ylthio)-5-nitrophthalsäuredinitril erhalten, das nach Auflösen in Aceton/Methylenchlorid, Filtrieren der Lösung über Kieselgel und Eindampfen des Filtrates 1,17 g (47% d. Theorie) reines Produkt ergibt.

| Elementaranalyse: C₁₁H₅N₅O₂S (MG 271,26): | | | | | |
|---|---|---|---|---|---|
| Ber.: | C 48,71 | H 1,86 | N 25,82 | O 11,80 | S 11,82 |
| Gef.: | C 48,86 | H 2,02 | N 25,56 | O 12,18 | S 11,63 |

Massenspektrum (m/e): 271 (M⁺; Basispeak)

Beim Erhitzen färbt sich die Substanz gelblich, dann olivgrün bis schwarz.

### Beispiel A10: 3-(Imidazol-2-ylthio)-5-methylthiophthalsäuredinitril

Einer Lösung von 0,40 g (1,47 mMol)3-(Imidazol-2-ylthio)-5-nitrophthalsäuredinitril in 5 ml DMSO werden bei 15°C 0,10 g (1,47 mMol) Natriummethylmercaptid zugesetzt.Das Reaktionsgemisch wird 15 MInuten bei 15°C gerührt und dann in eine Lösung von 1 ml Eisessig in 30 ml Wasser ausgetragen. Die entstandene Emulsion wird mit Tetrahydrofuran/Toluol extrahiert, die vereinigten Extrakte mit Wasser gewaschen, über Na₂SO₄ getrocknet und bei einer 60 °C nicht überschreitenden Temperatur eingedampft. Der Rückstand (0,38 g; 95% d. Theorie) wird in 15% Aceton/85% Methylenchlorid gelöst und über Kieselgel chromatographiert. Durch Eindampfen der Produktfraktionen und Umkristallisation aus Tetrahydrofuran/Toluol werden 0,18 g (45% d. Theorie) reines 3-(Imidazol-2-yl-thio)-5-nitrophthalsäuredinitril erhalten.

MS (M/e): 272 (M⁺; Basispeak)

Beim Erhitzen färbt sich die Substanz zunächst grünlichbraun und dann schwarz.

### Beispiel A11: 3-(Imidazol-2-ylthio)-5-isobutylthiophthalsäuredinitril

0,92 g (9,17 mMol) 2-Mercaptoimidazol, 5,07 g (36,7 mMol) K₂CO₃ und 15 ml Tetrahydrofuran werden unter Rühren auf -8°C abgekühlt. Dann wird eine Lösung von 2,0 g (9,17 mMol) 3,5-Dinitrophthalsäuredinitril in 7 ml Tetrahydrofuran unter Rühren und Kühlen langsam zugetropft und 30 Minuten bei 0-5°C gerührt. Dann wird das gebildete 3-(Imidazol-2-ylthio)-5-nitrophthalsäuredinitril durch Eintropfen einer Lösung von 0,99 g (11,01 mMol) Isobutylmercaptan und Nachrühren während 20 Minuten bei 5°C unmittelbar weiter umgesetzt. Danach wird das Reaktionsgemisch in eine Lösung von 7 ml Eisessig in 200 ml Wasser ausgetragen und mit Methylenchlorid extrahiert. Die vereinigten Exrakte werden über Na₂SO₄ getrocknet und mit Methylenchlorid über Kieselgel chromatographiert. Das erhaltene Rohprodukt (0,5 g; 20% d. Theorie) wird aus Diethylether umkristallisiert. Es werden 0,12 g (4% d. Theorie) 3-(Imidazol-2-ylthio)-5-isobutylthiophthalsäuredinitril erhalten.

MS (m/e): 314 (M⁺); 258 (Basispeak)

Beim Erhitzen zersetzt sich die Substanz unter Gelb-, Grün- und schliesslich Schwarzfärbung.

### Beispiel A12: 3-(Imidazol-2-ylthio)-5-phenylthiophthalsäuredinitril

Einer Lösung von 0,40 g (1,47 mMol) 3-(Imidazol-2-ylthio)-5-nitrophthalsäuredinitril in 5 ml DMSO werden bei 15°C 0,20 g (1,77 mMol) Thiophenol und 0,51 g (3,69 mMol) K₂CO₃ zugesetzt. Das Reaktionsgemisch wird 15 Minuten bei 15°C gerührt und dann in eine Lösung von 1 ml Eisessig in 30 ml Wasser ausgetragen. Die entstandene Emulsion wird mit Tetrahydrofuran/Toluol extrahiert, die vereinigten Extrakte mit Wasser gewaschen, über Na₂SO₄ getrocknet und bei einer 60°C nich überschreitenden Temperatur im Vakuum eingedampft. Der Rückstand wird mit 15% Aceton in Methylenchlorid an Kieselgel chromatographiert. Durch Eindampfen der Produktfraktionen werden 0,15 g (31% d. Theorie) reines 3-(Imidazol-2-ylthio)-5-phenylthiophthalsäuredinitril erhalten.

MS (m/e): 334 (M⁺; Basispeak).

Die Substanz färbt sich beim Erhittzen bräunlich-oliv, dann olivgrün und schliesslich schwarz.

### Beispiel A13:

3-(Imidazol-2-ylthio)-5-(3-methoxyphenylthio)-phthalsäuredinitril 0,92 g (9,17 mMol) 2-Mercaptoimidazol, 5,07 g (36,7 mMol) K₂CO₃ und 15 ml Tetrahydrofuran werden unter Rühren auf 0°C abgekühlt. Dann wird bei 0°C während 10 Minuten eine Lösung von 2,0 g 3,5-Dinitrophthalsäuredinitril in 7 ml Tetrahydrofuran zugetropft. Das Reaktionsgemisch wird 15 Minuten bei 0°C gerührt. Das gebildete 3-(Imidazol-2-ylthio)-5-nitrophthalsäuredinitril wird durch Zutropfen einer Lösung von 1,42 g (10,09 mMol) 3-Methoxythiophenol unmittelbar weiter umgesetzt. Nach beendigter Zugabe wird 10 minuten bei 0°C nachgerührt. Dann wird das Reaktionsgemisch in eine Lösung von 7 ml Eisessig in 200 ml Wasser ausgetragen und mit Methylenchlorid extrahiert. Die vereinigten Extrakte werden über Na₂SO₄ getrocknet und bei maximal 40°C eingedampft. Nach Chromatographieren des Rückstandes mit 15% Aceton in Methylenchlorid und Umkristallisieren aus Diethylether werden 0,59 g (18% d. Theorie) reines 3-(Imidazol-2-yl-thio)-5-phenylthiophthalsäuredinitril erhalten.

MS (m/e): 364 (M⁺; Basispeak).

Beim Erhitzen färbt sich die Substanz gelbgrün, dann grün und schliesslich schwarz.

### Beispiel A14:

### Verbindung Nr. 1

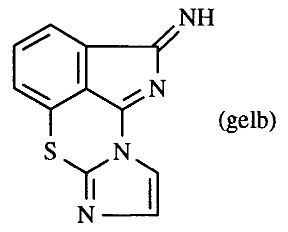

2,50 g (11,05 mMol) 3-(Imidazol-2-ylthio)-phthalsäuredinitril werden in 200 ml o-Dichlorbenzol während 2 Stunden unter Rückfluss erhitzt.Dann wird das Gemisch abgekühlt und mit Methylenchlorid und steigenden Mengen Aceton an Kieselgel chromatographiert. Die Produktfraktionen werden eingedampft und der Rückstand (1,45 g; 28% d. Theorie) bei 190°C/1 Torr sublimiert. Als Sublimat werden 0,71 g (28% d. Theorie) reine Verbindung 1 erhalten.

MS (m/e): 226 (M⁺; Basispeak).

Das NMR-Spektrum zeigt ein Gemisch von syn/anti-NH 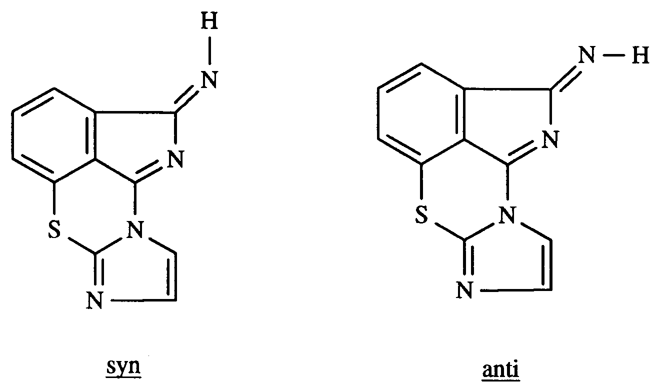
im Verhältnis von ≅ 3:2 oder 2:3.

UV/VIS-Spektrum (λₘₐₓ/ε; DMF): 386/5200

Das IR-Spektrum (KBr; CH₂Cl₂) zeigt keine Nitrilgruppen.

Die Verbindung verfärbt sich beim Erhitzen zunächst gelb, dann grün und schliesslich schwarz.

### Beispiel A15:

### Verbindung Nr. 2

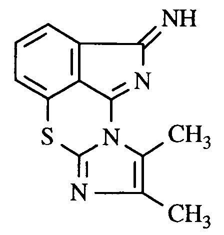

Nach der in Beispiel A14 beschriebenen Arbeitsweise wird durch 4-stündiges Erhitzen von 3-(3,4-Dimethylimidazol-2-ylthio)-phthalsäuredinitril in Xylol unter Rückfluss ein syn/anti-Gemisch der Verbindung 2 erhalten. Ausbeute: 29% d. Theorie; MS (m/e): 254 (M⁺; Basispeak).

Die Verbindung verfärbt sich beim Erhitzen zunächst gelb, dann grün und schliesslich schwarz.

### Beispiel A16:

### Verbindung Nr. 3

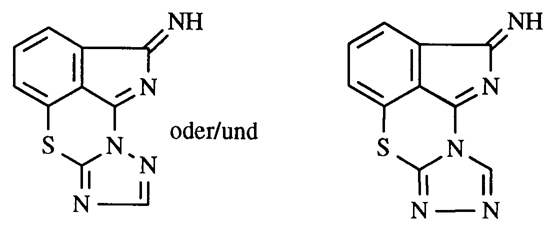

(vermutlich Hauptkomponente)

Nach der in Beispiel A14 beschriebenen Arbeitsweise wird durch 4-stündiges Erhitzen von 3-(1(H)-1,2,4-Triazol-5-ylthio)-phthalsäuredinitril in Mesitylen unter Rückfluss ein syn/anti-Gemisch der Verbindung 3 erhalten. Ausbeute: 14% d. Theorie; MS (m/e): 227 (M⁺; Basispeak).

Die Verbindung verfärbt sich beim Erhitzen zunächst gelb, dann grün und schliesslich schwarz.

### Beispiel A17:

### Verbindung Nr. 4

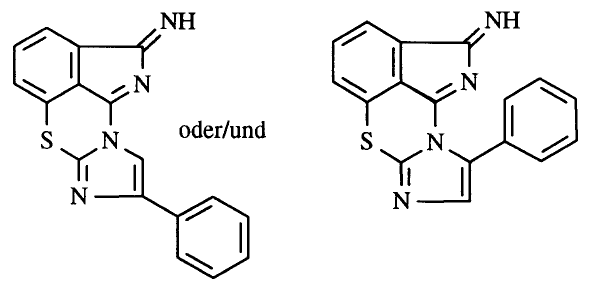

(vermutlich Hauptkomponente)

Nach der in Beispiel 7 beschriebenen Arbeitsweise wird durch 9-stündiges Erhitzen von 3-(4-Phenylimidazol-2-ylthio)-phthalsäuredinitril in Mesitylen unter Rückfluss ein syn/anti-Gemisch der Verbindung 4 erhalten. Ausbeute: 23% d. Theorie; MS (m/e): 302 (M⁺; Basispeak).

Die Verbindung verfärbt sich beim Erhitzen zunächst gelb, dann grün und schliesslich schwarz.

### B) Anwendungsbeispiele

### Beispiel B1:

Eine 3,5 Gew.%ige Lösung von 3-(Imidazol-2-ylthio)-5-phenylthiophthalsäuredinitril (vgl. Beispiel A12) in Tetrahydrofuran wird durch Schleuderbeschichtung bei 250 U/min auf einen Glasträger aufgetragen. Nach dem Trocknen beträgt die Schichtdicke des Feststoffilmes 0,42 µm. Der gelbliche Film zeigt im Wellenlängenbereich von 400 nm - 900 nm eine geringe Absorption (dekadischer Absorptionskoeffizient <0,1). Nach 5 Minuten Tempern bei 250°C bildet sich eine amorphe, dunkelolive Schicht, die im erwähnten Wellenlängenbereich einen sehr breitbandigen Absorptionsverlauf zeigt (maximaler Absorptionskoeffizient 0,8).

### Beispiel B2:

Eine 5 Gew.%ige Lösung von 3-(Imidazol-2-ylthio)-5-isobutylthiophthalsäuredinitril (vgl. Beispiel A11) wird durch Schleuderbeschichtung auf einen Glasträger aufgetragen. Die getrocknete Feststoffschicht zeigt im Wellenlängenbereich von 500 nm - 900 nm eine kleine Absorption (dekadischer Absorptionskoeffizient <0,05). Nach einer Temperaturbehandlung während 15 Minuten bei 200°C verändert sich die Farbe von gelb zu dunkelolive mit starker Absorption im sichtbaren und im nahen Infrarot-Bereich mit einem Absorptionsmaximum bei 680 nm.

## Patentansprüche

1. Verbindungen der Formel I 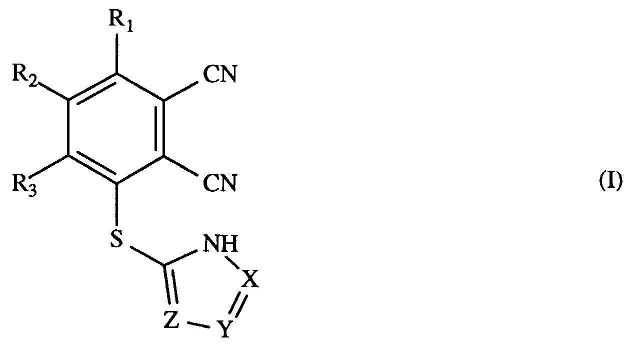 in welcher R₁, R₂ und R₃ unabhängig voneinander je Wasserstoff, eine geradkettige oder verzweigte C₁-C₁₂-Alkylgruppe, die unsubstituiert oder mit Alkoxy, Alkylthio, Aryloxy, Arylthio, Halogen, -CN, -COOH, -COOR₇, -NR₈R₉ oder -CONR₈R₉ substituiert ist, eine C₁-C₁₂-Alkoxygruppe, in der die Alkylgruppe geradkettig oder verzweigt und unsubstituiert oder mit Alkoxy, Alkylthio, Aryloxy, Arylthio-, Halogen, -CN, -COOH, -COOR₇, -NR₈R₉ oder -CONR₈R₉ substituiert ist, eine C₁-C₁₂-Alkylthiogruppe, in der die Alkylgruppe geradkettig oder verzweigt und unsubstituiert oder mit Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkoxygruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₅-C₈-Cycloalkylthiogruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₆-C₁₀-Arylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, eine C₆-C₁₀-Aryloxygruppe, in der die Arylgruppe unsubstituiert oder mit Alkyl, Alkoxy, Alkythio, Phenyl, Phenoxy, Phenylthio, Halogen, -CN, -COOH, -COOR₇, -NR₈R₉ oder -CONR₈R₉ substituiert ist, eine C₆-C₁₀-Arylthiogruppe, in der die Arylgruppe unsubstituiert oder mit Alkyl, Alkoxy, Alkylthio, Phenyl, Phenoxy, Phenylthio, Halogen, -CN, -COOH, -COOR₇, -NR₈R₉ oder -CONR₈R₉ substituiert ist, Nitro, Halogen, Cyano, -COOH, -COOR₇, -NR₈R₉ oder -CONR₈R₉ bedeuten, wobei R₇ C₁-C₂₀-Alkyl, C₅-C₈-Cycloalkyl, Phenyl oder Phenyl-C₁-C₄-alkyl bedeutet, und R₈ und R₉ unabhängig voneinander je Wasserstoff, C₁-C₈-Alkyl, Phenyl oder Phenyl-C₁-C₄-alkyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, eine geradkettige oder verzweigte C₁-C₁₂-Alkylgruppe, die unsubstituiert oder mit Alkoxy, Alkylthio, Aryloxy, Arylthio, Halogen, -CN, -COOH, -COOR₇, -NR₈R₉ oder -CONR₈R₉ substituiert ist oder eine C₆-C₁₀ -Arylgruppe, die unsubstituiert oder mit Alkyl, Alkoxy, Alkylthio, Aryloxy, Arylthio, Halogen, -CN, -COOH, -COOR₇, -NR₈R₉ oder -CONR₈R₉ substituiert ist, bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen eine Gruppe 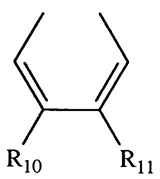 oder eine Tetramethylengruppe bilden, wobei R₁₀ und R₁₁ unabhängig voneinander Wasserstoff, C₁-C₆-Alkyl, Phenyl oder Phenyl-C₁-C₄-alkyl darstellen.

2. Verbindungen nach Anspruch 1, dadurch gekennzeichnet, dass die Reste R₁, R₂ und R₃ unabhängig voneinander je Wasserstoff, eine C₁-C₈-Alkylgruppe, die unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₈-Alkoxygruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, NR₈R₉ oder -CONR₈R₉ substituiert ist, eine C₁-C₈-Alkylthiogruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyl- oder Naphthylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyloxy oder Naphthyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenylthio-, oder Naphthylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, Fluor, Chlor, Brom, Nitro, Cyano, -COOH, -COOR₆, -NR₇R₈ oder -CONR₇R₈ bedeuten, wobei R₇ C₁-C₈-Alkyl oder Phenyl bedeutet und R₈ und R₉ unabhängig voneinander je Wasserstoff, C₁-C₈-Alkyl, Phenyl oder Phenyl-C₁-C₄-alkyl darstellen.

3. Verbindungen nach Anspruch 2, dadurch gekennzeichnet, dass die Reste R₁, R₂ und R₃ unabhängig voneinander je Wasserstoff, eine C₁-C₄-Alkylgruppe, die unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₄-Alkoxygruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine C₁-C₄-Alkylthiogruppe, in der die Alkylgruppe geradkettig oder verzweigt und unsubstituiert oder mit C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Cyclohexylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyl-oder Naphthylgruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenyloxy- oder Naphthyloxygruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, eine Phenylthio- oder Naphthylthiogruppe, die unsubstituiert oder mit C₁-C₄-Alkyl, C₁-C₄-Alkoxy, C₁-C₄-Alkylthio, Phenyl, Phenoxy, Phenylthio, Fluor, Chlor, Brom, -CN, -COOH, -COOR₇, -NR₈R₉ oder -CONR₈R₉ substituiert ist, Fluor, Chlor, Brom, Nitro, Cyano, -COOH, -COOR₆, -NR₇R₈ oder -CONR₇R₈ bedeuten, wobei R₇ C₁-C₄-Alkyl oder Phenyl bedeutet und R₈ und R₉ unabhängig voneinander je Wasserstoff, C₁-C₄-Alkyl, Phenyl oder Benzyl darstellen.

4. Verbindungen nach Anspruch 1, dadurch gekennzeichnet, dass die in den Verbindungen der Formel I enthaltene Azolylgruppe der Formel 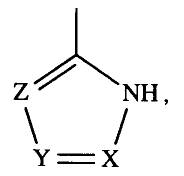 wobei X Stickstoff oder CR₄, Y Stickstoff oder CR₅ und Z Stickstoff oder CR₆ bedeuten und R₄, R₅ und R₆ die im Anspruch 1 angegebene Bedeutung haben, unsubstituiertes oder mit R₄, R₅, R₆ substituiertes Pyrrol-2-yl-, Imidazol-2-yl-, Imidazol-4-yl-, Imidazol-5-yl-Pyrazol-3-yl-, Pyrazol-5-yl-, 1H-1,2,3-Triazol-5-yl-, 2H-1,2,3-Triazol-5-yl-, 3H-1,2,3-Triazol-5-yl-, 1H-1,2,4-Triazol-5-yl-, 2H-1,2,4-Triazol-2-yl-, 4H-1,2,4-Triazol-2-yl-, 1H-1,2,3,4-Tetrazol-5-yl-, 2H-1,2,3,4-Tetrazol-5-yl-, Indol-2-yl-, Isoindol-2-yl-, Benzimidazol-2-yl-, Benzpyrazol-3-yl-, 4,5,6,7 Tetarhydro-Indol-2-yl-, 4,5,6,7 Tetrahydro-Isoindol-2-yl-, 4,5,6,7 Tetrahydro-Benzimidazol-2-yl-, 4,5,6,7 Tetrahydro-Benzpyrazol-3-yl- darstellt, wobei die annellierten Benzolringe oder Tetrahydrobenzolringe durch die Reste R₁₀ und R₁₁ der in Anspruch 1 unter Formel I angegebenen Bedeutung substituiert sein können.

5. Verbindungen nach Anspruch 1, dadurch gekennzeichnet, dass X CR₄, Y CR₅ und Z CR₆ bedeuten, wobei R₄, R₅ und R₆ unabhängig voneinander je C₁-C₄-Alkyl-, C₁-C₄-Halogenalkyl oder Phenyl bedeuten oder R₄ und R₅ oder R₅ und R₆jeweils zusammen eine 1,3,-Butadienylengruppe darstellen.

6. Verbindungen nach Anspruch 1, dadurch gekennzeichnet, dass X CR₄, Y CR₅ und Z Stickstoff bedeutet, wobei R₄ und R₅ unabhängig voneinander je Wasserstoff, C₁-C₄-Alkyl, C₁-C₄-Halogenalkyl oder Phenyl bedeuten oder R₄ und R₅ zusammen einen 1,3-Butadienylenrest darstellen.

7. Verbindungen nach Anspruch 1, dadurch gekennzeichnet, dass X CR₄, Y Stickstoff und Z CR₆ bedeutet, wobei R₄ und R₆ unabhängig voneinander je Wasserstoff, C₁-C₄-Alkyl, C₁-C₄-Halogenalkyl oder Phenyl bedeuten.

8. Verbindungen nach Anspruch 1, dadurch gekennzeichnet, dass X Stickstoff, Y CR₅ und Z CR₆ bedeutet, wobei R₅ und R₆ unabhängig voneinander je Wasserstoff, C₁-C₄-Alkyl, C₁-C₄-Halogenalkyl oder Phenyl bedeuten oder R₅ und R₆ zusammen einen 1,3-Butadienylenrest darstellen.

9. Verbindungen nach Anspruch 1, dadurch gekennzeichnet, dass X und Y Stickstoff bedeuten und Z CR₆ ist, wobei R₆ Wasserstoff, C₁-C₄-Alkyl, C₁-C₄-Halogenalkyl oder Phenyl bedeutet.

10. Verbindungen nach Anspruch 1, dadurch gekennzeichnet, dass X, Y und Z Stickstoff bedeuten.

11. Verbindungen nach Anspruch 1, dadurch gekennzeichnet, dass R₁ und R₃ Wasserstoff bedeuten und R₂, X, Y und Z die in Anspruch 1 unter Formel I angegebene Bedeutung haben.

12. Verbindungen nach Anspruch 1, dadurch gekennzeichnet, dass R₁ und R₃ Wasserstoff, R₂ C₁-C₈-Alkyl, C₁-C₈-Alkoxy, C₁-C₈-Alkylthio, Cyclohexyl, Cyclohexyloxy, Cyclohexylthio, Phenyl, Phenoxy, Phenylthio, Fluor, Chlor, Brom, Nitro, Cyano, -COOH, -COOR₇, -NR₈R₉ oder -CONR₈R₉ bedeuten, wobei R₇ C₁-C₈-Alkyl, Phenyl-C₁-C₄-alkyl oder Phenyl bedeutet und R₈ und R₉ unabhängig voneinander je Wasserstoff, C₁-C₈-Alkyl, Phenyl oder Phenyl-C₁-C₄-alkyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, C₁-C₄-Alkyl, C₁-C₄-Halogenalkyl oder Phenyl bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen auch eine 1,3-Butadienylengruppe bilden können.

13. Verbindungen nach Anspruch 1, dadurch gekennzeichnet, dass R₁ und R₃ Wasserstoff, R₂ C₁-C₄-Alkyl, C₁-C₄-Alkoxy, C₁-C₄-Alkylthio, Cyclohexyl Cyclohexyloxy, Cyclohexylthio, Phenyl, Phenoxy, Phenylthio, Fluor, Chlor, Brom, Nitro, Cyano, -COOH, -COOR₇, -NR₈R₉ oder -CONR₈R₉ bedeuten, wobei R₇ C₁-C₄-Alkyl, Phenyl Benzyl bedeutet, R₈ und R₉ unabhängig voneinander je Wasserstoff, C₁-C₄-Alkyl, Phenyl oder Benzyl darstellen, X Stickstoff oder CR₄, Y Stickstoff oder CR₅ und Z Stickstoff oder CR₆ bedeuten, wobei R₄, R₅ und R₆ unabhängig voneinander je Wasserstoff, C₁-C₄-Alkyl, C₁-C₄-Halogenalkyl oder Phenyl bedeuten und wobei, wenn X CR₄ und Y CR₅ bedeuten und Z für Stickstoff oder CR₆ steht, oder wenn Y CR₅ und Z CR₆ bedeuten und X für Stickstoff oder CR₄ steht, R₄ und R₅ bzw. R₅ und R₆ jeweils zusammen auch eine 1,3-Butadienylengruppe bilden können.

14. Verbindungen nach Anspruch 1, dadurch gekennzeichnet, dass R₁ und R₃ Wasserstoff bedeuten, R₂ für Wasserstoff, Nitro, C₁-C₄-Alkylthio oder Phenylthio steht, wobei die Phenylgruppe durch C₁-C₄-Alkyl, C₁-C₄-Alkoxy oder C₁-C₄-Alkylthio substituiert sein kann, X CR₄, Y CR₅ oder Stickstoff und Z Stickstoff bedeutet, wobei R₄ und R₅ unabhängig voneinander Wasserstoff, C₁-C₄-Alkyl, C₁-C₄-Halogenalkyl, Phenyl oder zusammen eine Gruppe 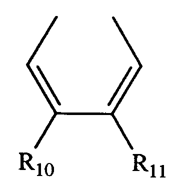 darstellen, in welcher R₁₀ und R₁₁ unabhängig voneinander Wasserstoff oder C₁-C₄-Alkyl bedeuten.

15. Verbindungen der Formel IV, 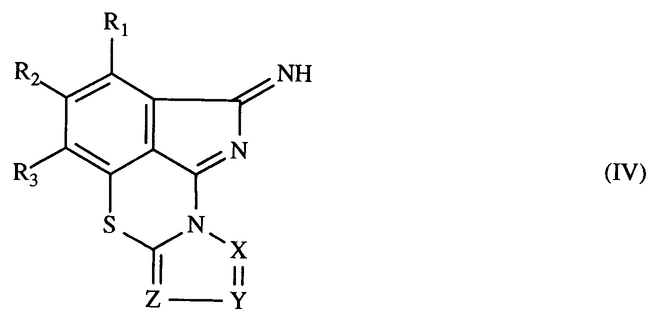 in welcher R₁, R₂, R₃, X, Y und Z die unter Formel I angegebene Bedeutung haben.

16. Verfahren zur Herstellung von Verbindungen der I, 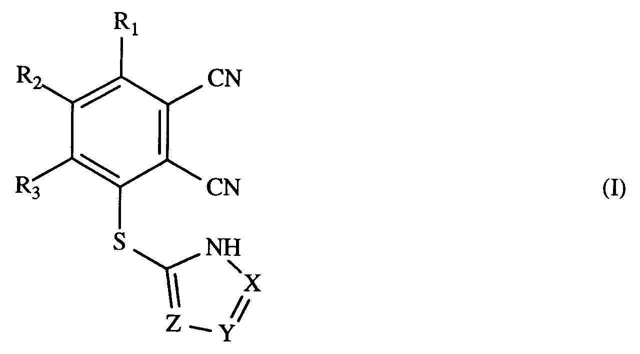 in welcher R₁, R₂, R₃, X, Y und Z die in Anspruch I unter Formel I angegebenen Bedeutungen haben, dadurch gekennzeichnet, dass man ein substituiertes Phthalsäuredinitril der Formel II 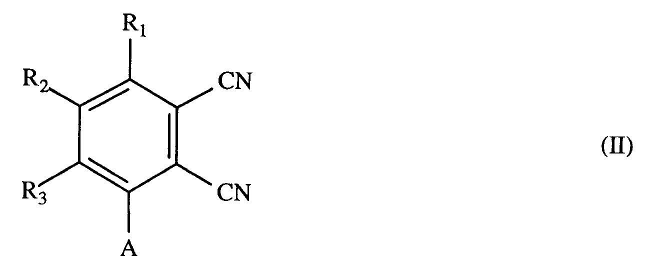 in welcher R₁, R₂ und R₃ die unter Formel I angegebenen Bedeutungen haben und A für Halogen oder eine Nitrogruppe steht, in einem inerten Lösungsmittel in Gegenwart einer Base mit einem Azol der allgemeinen Formel III 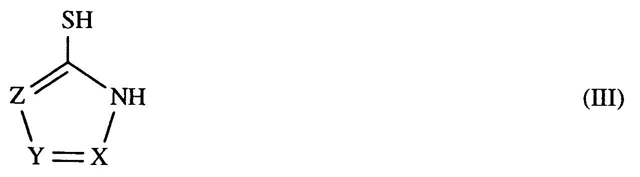 in welcher X, Y und Z die unter Formel I angegebenen Bedeutungen haben, umsetzt.

17. Verfahren zur Herstellung von Verbindungen der Formel IV, 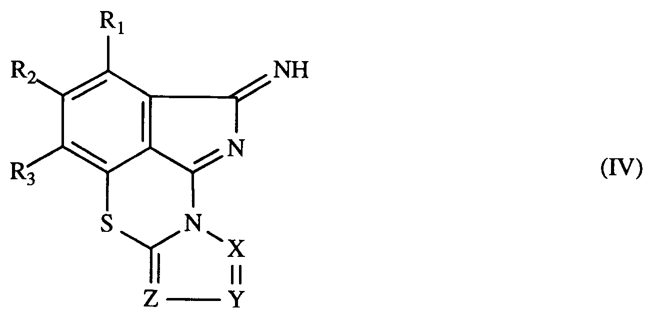 in welcher R₁, R₂, R₃, X, Y und Z die in Anspruch 1 unter Formel I angegebenen Bedeutungen haben, dadurch gekennzeichnet, dass man man eine Verbindung der Formel I 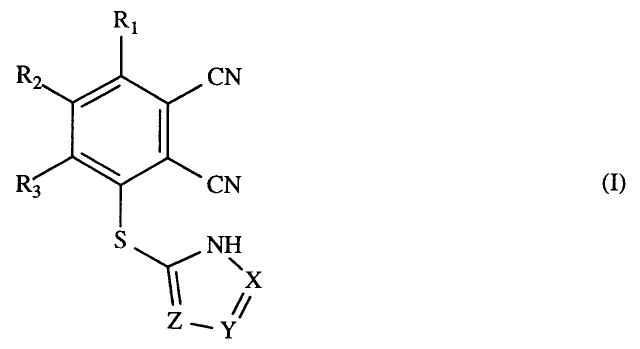 in welcher R₁, R₂, R₃, X, Y und Z die in Anspruch 1 unter Formel I angegebenen Bedeutungen haben, auf eine Temperatur im Bereich von 75°C bis 300°C, vorzugsweise 90°C bis 160°C, erhitzt.

18. Thermochrome Zusammensetzung, die als thermochrome Komponente mindestens eine Verbindung der Formel I gemäss Anspruch 1 und/oder mindestens eine Verbindung der Formel IV gemäss Anspruch 15 enthält.

19. Thermochrome Zusammensetzung nach Anspruch 18, dadurch gekennzeichnet, dass die aus mindestens einer Verbindung der Formel I gemäss Anspruch 1 und/oder mindestens einer Verbindung der Formel IV gemäss Anspruch 15 bestehende thermochrome Komponente in einer Menge von 0,001 bis 20 Gew.% bezogen auf die Zusammensetzung enthalten ist.

20. Thermochrome Zusammensetzung, enthaltend
a) ein farbloses organisches Lösungsmittel, ein Polymer oder ein organisches Glas und
b) gelöst, eingemischt oder als Schicht auf mindestens einer Oberfläche mindestens eine Verbindung der Formel I gemäss Anspruch 1 und/oder mindestens eine Verbindung der Formel IV gemäss Anspruch 15.

21. Thermochrome Zusammensetzung nach Anspruch 20, dadurch gekennzeichnet, dass die Komponente b) bezogen auf Komponente a) in einer Menge von 0,001 bis 20 Gew.% enthalten ist.

22. Material zur Aufzeichnung und zur Speicherung von Informationen, bei dem auf einem Träger mindestens eine Schicht mit mindestens einer der Verbindungen der Formeln I und IV gegebenenfalls zusammen mit einem Bindemittel als speicheraktive Schicht aufgebracht ist.

23. Material gemäss Anspruch 22, das den Aufbau:
(a) transparenter Träger, (b) Aufzeichnungsschicht und (c) transparente Schutzschicht;
oder
(a) transparenter Träger, (b) Aufzeichnungsschicht, (c) Reflexionsschicht und (d) Schutzschicht;
oder
(a)Träger, (b) Reflexionsschicht, (c) Aufzeichnungsschicht und (d) transparente Schutzschicht, besitzt.

24. Verfahren zum bildhaften Aufzeichnen, bevorzugt optischen Aufzeichnen und Speichern von Informationen in Form von Bits, bei dem man ein erfindungsgemässes Aufzeichnungsmaterial bildmässig Wärme oder einer Wärmestrahlung, oder punktweise oder linienweise einer Laserstrahlung im NIR-Bereich aussetzt.

25. Informationen enthaltendes Material, bei dem in der Aufzeichnungsschicht eines Aufzeichnungmaterials gemäss Anspruch 22 Bilder oder Markierungen in Form von Bits aufgebracht bzw. eingeschrieben sind, die im nahen Infrarotbereich gegenüber der unveränderten Umgebung eine verminderte Reflexion und erhöhte Absorption aufweisen.

26. Verwendung von Verbindungen der Formeln I oder IV als irreversible thermochrome Systeme zur Kontrastbildung, Lichtabsorption und als thermochrome Farbindikatoren.

27. Verwendung der Verbindung der Formel I oder IV zur irreversiblen optischen Speicherung von Information, wobei die Information in einer die Verbindung enthaltenden, speicheraktiven Schicht mit Wärmestrahlung, bevorzugt mit IR-Laserstrahlen, eingeschrieben wird.

28. Verwendung der Verbindung der Formel I oder IV als Aktivbestandteil in thermochromen Anzeigeelementen.

29. Verwendung der Verbindungen der Formel I oder IV als thermochromer Aktivbestandteil für Bilderzeugungsverfahren unter Einwirkung von Wärme oder Wärmestrahlung.
